# EUROPEAN PATENT APPLICATION

(11) **EP 4 609 884 A1**
(43) Date of publication of application: **03.09.2025**
(21) Application number: 24160174.9
(22) Date of filing: 28.02.2024
(51) Int. Cl.: A61L 27/06, A61F 2/28, A61F 2/30

(54) **A TITANIUM PROSTHETIC IMPLANT**

(71) Applicant: Elos Medtech Pinol A/S, 3330 Gørløse (DK)
(72) Inventor: Bojsen-Møller, Frederik, Gørløse (DK); Grüner, Magnus Felix, Gørløse (DK); Nyholm-Petersen, Daniel Kim, Gørløse (DK); Olsen, Søren, Gørløse (DK); Andersen, Henrik, Gørløse (DK)
(74) Representative: Plougmann Vingtoft a/s

(57) **Abstract**

The present invention relates to a titanium prosthetic implant, a titanium prosthetic implant system and a method of reducing wear and abrasion during use of a titanium prosthetic implant.

## Description

### FIELD OF THE INVENTION

The present invention relates to a titanium prosthetic implant, a titanium prosthetic implant system and a method of reducing wear and abrasion during use of a titanium prosthetic implant.

### BACKGROUND OF THE INVENTION

A prosthetic implant refers to an artificial device or structure that is surgically implanted into the body to replace or augment a missing or damaged part. These implants are designed to restore function, enhance mobility, or improve the quality of life for individuals who have lost a body part or have a significant impairment. Prosthetic implants can be used for various purposes, such as replacing joints (e.g., hip or knee replacements), limbs (e.g., artificial limbs or prosthetic arms/legs), dental implants, and other body parts.

These implants are typically made from biocompatible materials, such as metals (like titanium), ceramics, or polymers, to ensure compatibility with the human body and minimize the risk of rejection or adverse reactions. The design and materials used for a prosthetic implant depend on the specific function it is intended to serve and the anatomical location in the body where it will be implanted.

In use, prosthetic implants surfaces are generally in contact with other polymers surfaces in prosthetic implants systems.

The interface between a prosthetic implant and polymer is carefully designed to optimize biocompatibility, mechanical properties, and overall performance. These considerations are crucial for ensuring the success and longevity of the implant while minimizing the risk of complications or adverse reactions in the patient.

The most common problem associated with the prosthetic implant/polymer interface includes wear and abrasion. In general, over time, repetitive motion or friction between the polymer components of the implant and adjacent materials can lead to wear and abrasion.

This can result in the release of wear debris, potentially causing inflammation, tissue damage, and implant failure.

In this context, hence, a prosthetic implant with increased wear resistance ensuring the longevity of the implant and reducing the generation of wear debris that could potentially lead to inflammatory reactions or implant failure would be advantageous.

### OBJECT OF THE INVENTION

An object of the present invention is to provide a prosthetic implant and a prosthetic implant system reducing the probability of implant failure, diminished functionality, and potential complications during its use.

A further object of the present invention is also to provide a prosthetic implant and a prosthetic implant system supplying a durable and long-lasting solution for patients undergoing joint replacement or other orthopedic interventions.

In particular, it may be seen as an object of the present invention to provide a prosthetic implant mitigating wear and abrasion by producing a metallic surface layer with improved hardness with minimal brittleness and roughness.

An object of present invention is also to implement a method for reduce wear and abrasion in a prosthetic implant system to enhance its longevity.

An even further object of the invention may also be seen as to provide an alternative to the prior art.

### SUMMARY OF THE INVENTION

The idea of the invention is to provide a titanium prosthetic implant with high resistance to wear and abrasion during use in a titanium prosthetic implant system.

In the first aspect, the invention relates to a titanium metal or titanium metal alloy prosthetic implant comprising a metallic surface layer having a thickness between 5 and 50 µm and a surface hardness above 700 HV_{0.05}.

In some embodiments, the metallic surface layer has a surface roughness in the range between 0.1 and 0.01 Ra.

The metallic surface layer assessed through a VDI 3198 indentation test shows a hardness factor (HF) of 1 (HF1).

The titanium prosthetic implant of the invention may be a joint replacement implant, such as a hip or knee replacement.

The titanium prosthetic implant of the invention is characterized by a titanium metallic surface layer, such as a surface composed of hexagonal closed packed (HCP) titanium (Ti) detectable by X-Ray Diffraction (XRD), without the presence of nitrides or oxides, apart from the 3 - 10 nm TiOz passivation layer that always forms on Ti. This hardened layer, extending from the surface and about 5 to 50 µm into the surface, is formed through a case-hardening process, i.e., a process providing hardness only on the external surface layer, leaving the core relatively softer.

The titanium prosthetic implant of the invention exhibits a hard metallic surface layer with a hardness gradient, i.e., a tough and ductile core with a hard and wear-resistant surface.

The metallic surface layer has a surface hardness of above 700 HV_{0.05}, such as between 800 and 1300 HV_{0.05}, detectable through a Vickers hardness test based on the mean value of three indentations set with a load of 50g and a dwell time of 10s.

The metallic surface layer has a surface roughness in the range between 0.1 and 0.01 Ra, being Ra the average roughness, i.e., the arithmetic average of the absolute values of the roughness profile deviations from the mean line within a standard evaluation length. Surface roughness may be measured using a standard profilometer apparatus.

The metallic surface layer of the invention shows a hardness factor (HF) of 1 (HF1) as detectable through a Rockwell C indentation interpreted according to VDI 3198 guideline.

In search for solution for reducing wear and abrasion in titanium prosthetic implant the inventors devised the invention by producing a metallic surface layer having a combination of hardness, roughness and brittleness which synergically provide unique wear and abrasion reduction properties for application in this particular technical field.

In some embodiments, the metallic surface layer is produced by case hardening of the titanium metal or a titanium metal alloy prosthetic implant.

Case hardening selectively targets the surface layer to achieve localized hardness, leaving the core with different mechanical properties.

In some embodiments, the case hardening is achieved by exposure to heat in a reactive atmosphere, such as in presence of COz or NH₃ gas.

The case-hardening process is performed in an environment with controlled atmospheres to prevent the oxidation of the metal surface. Oxygen can lead to the formation of oxides on the metal surface, negatively impacting the effectiveness of the case-hardening treatment.

The use of reactive atmosphere implies the use of reactive gases, such as NH₃ or COz and does not exclude the presence of other gases, such as inert gases, for example N₂.

In some embodiments, the surface roughness between 0.1 and 0.01 Ra of the metallic surface layer is achieved by a polishing process, such as an electropolishing process.

Electropolishing is one possible polishing process used to enhance the smoothness of the metallic surface layer of the titanium prosthetic implant of the invention.

In the electropolishing process the titanium prosthetic implant is immersed in an electrolyte solution, typically containing acids, while an electric current is applied. The electric current selectively dissolves microscopic peaks from the metal surface, resulting in the removal of surface imperfections, such as burrs, oxides, and contaminants.

In doing so, through electropolishing, the metallic surface layer is smoothened while rendering the external surface rather uniform.

Electropolishing is, generally, achieved by applying voltage to the system strictly according to the indication the producer of the electropolishing cell or electropolishing system.

In some embodiments, the roughness between 0.1 and 0.01 Ra of the metallic surface layer is achieved by applying a voltage of at least 40 V, such as between 40 V and 60 V, for example between 40 V and 50 V.

In a second aspect, the invention relates to a titanium metal alloy prosthetic implant system comprising the titanium metal or a titanium metal alloy prosthetic implant according to the first aspect of the invention and a polymer-based bearing component.

The titanium metal or titanium metal alloy prosthetic implant system is used in joint replacement surgeries, such as hip and knee replacements.

The polymer-based bearing component is often used in the articulating surfaces of the prosthetic joint, serving as the interface that comes into contact with the opposing joint surface. Common polymers for this application include ultra-high-molecular-weight polyethylene (UHMWPE) due to its wear resistance, low friction, and biocompatibility.

The polymer-based bearing component, in contact with the titanium implant, is designed to minimize wear and friction.

The combination of a titanium implant and a polymer-based bearing component in the system according to the second aspect of the invention aims at providing long-term performance by reducing wear. The materials used withstand the mechanical demands of daily activities, while minimizing the potential for material degradation.

In a third aspect, the invention relates to a method of reducing wear and abrasion during use of a titanium metal or a titanium metal alloy prosthetic implant system according to the second aspect of the invention.

The method according to the third aspect comprises two steps: case hardening the titanium or a titanium metal alloy prosthetic implant; and polishing the case-hardened titanium metal or a titanium metal alloy prosthetic implant by electropolishing applying a voltage of at least 40 V.

The step of case hardening allows the formation of a metallic surface layer extending between 5 to 50 µm into the external surface of the prosthetic implant, having a hardness above 700 HV_{0.05}.

The step of polishing may comprise applying a voltage of at least 40 V, such as between 40 V and 60 V, between an electrode and a counter electrode for one or more pre-determined periods of time.

During the polishing or electropolishing step an electropolishing system is used.

Deviation from the manufacturer's specified voltage settings during the electropolishing step may not be intuitively apparent, as these parameters are tailored to achieve optimal results. In search for producing an optimal surface for application in prosthetic implantation, the inventors devised the invention by departing from manufacturer's recommended voltage settings for Ti and Ti alloys thus applying voltages of at least 40 V, such as between 40 V and 60 V, for example between 40 V and 50 V.

Unexpectedly, in straying from the prescribed voltage indications, the inventors produced a hard metallic surface layer having an optimal surface roughness, in relation to its application, being between 0.1 and 0.01 Ra and a low level of brittleness, with a HF of 1 (HF1), as detectable though a VDI 3198 indentation test.

The first, second and other aspects, embodiments and items of the present invention may each be combined with any of the other aspects, embodiments and items. These and other aspects of the invention will be apparent from and elucidated with reference to the embodiments described hereinafter.

### BRIEF DESCRIPTION OF THE FIGURES

The titanium prosthetic implant, the titanium prosthetic implant system and the method of reducing wear and abrasion according to the invention will now be described in more details with regard to the accompanying figures.

The figures show one way of implementing the present invention and are not to be construed as being limiting to other possible embodiments falling within the scope of the attached claim set.
Figure 1 shows a schematic view of a titanium prosthetic implant according to some embodiments of the invention.
Figure 2 shows a schematic cross section view of a titanium prosthetic implant according to some embodiments of the invention.
Figure 3 shows a schematic cross section view of a titanium prosthetic implant after the case hardening process, according to some embodiments of the invention.
Figure 4 shows a schematic cross section view of a titanium prosthetic implant after the polishing process, according to some embodiments of the invention.
Figures 5 and 6 show a grayscale map and topographical curves showing Ra values of a titanium prosthetic implant after the case hardening process before the polishing step.
Figures 7 and 8 show a grayscale map and topographical curves showing Ra values of a titanium prosthetic implant after the case hardening process after the polishing step.
Figure 9 is a plot showing hardness vs distance from the external surface of titanium prosthetic implants according to some embodiments of the invention.
Figure 10 is a microscopy image of a metallic surface of a titanium prosthetic implant, according to some embodiments of the invention, following a hardness test.
Figure 11 is a flow chart of a method of reducing wear and abrasion during use of a titanium metal or a titanium metal alloy prosthetic implant system, according to one aspect of the invention.

### DETAILED DESCRIPTION OF AN EMBODIMENT

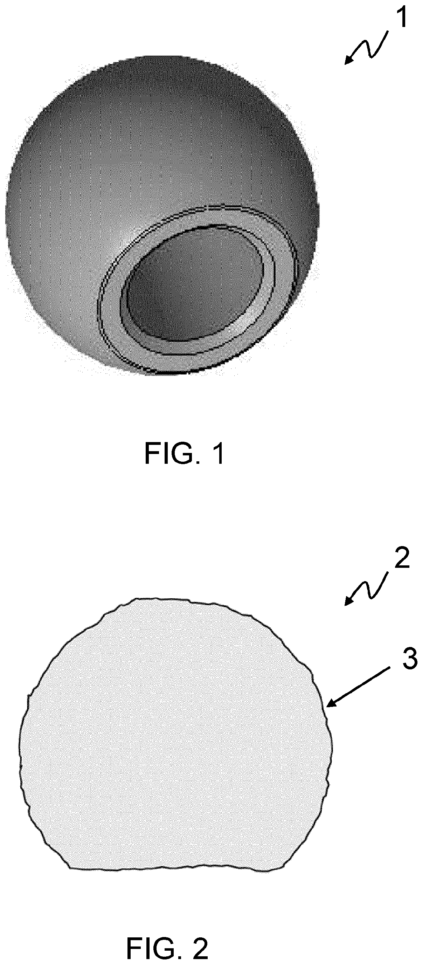

Figure 1 shows a schematic view of a titanium prosthetic implant 1 according to some embodiments of the invention.

Figure 2 shows a schematic cross section view of a titanium prosthetic implant 2 depicting a surface layer 3 having a surface roughness of the machined titanium implant or component in the area between 0.6 and 0.1 Ra.

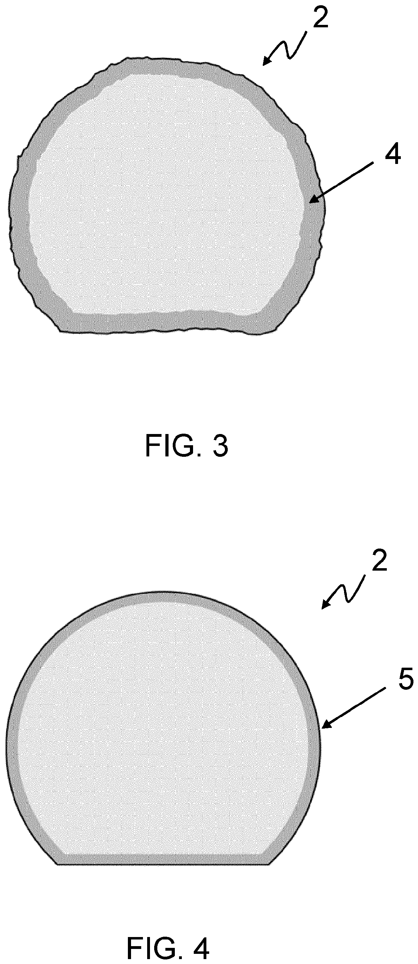

Figure 3 shows a schematic cross section view of the titanium prosthetic implant 2 of figure 2 after the case hardening process occurred.It can be noticed that, while the surface roughness is still in the area between 0.6 and 0.1 Ra, the case hardening process creates a metallic surface layer 4 having a thickness between 5 and 50 µm and a hardness above 700 HV_{0.05}.

Figure 4 shows a schematic cross section view of a titanium prosthetic implant 2 of figure 3 following the polishing process, realized through electropolishing by applying a voltage between 40 V and 50 V. It can be noticed that the surface roughness is depicted as being reduced to a value between 0.1 and 0.01, such as below 0.1 Ra, for example below 0.06 Ra.

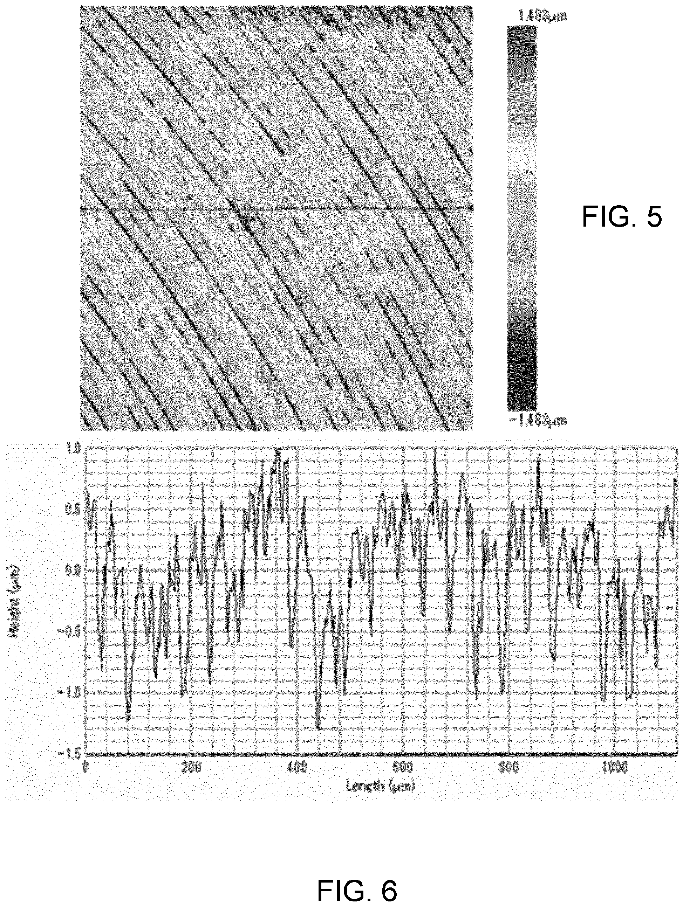

Figures 5 and 6 provide an indication of surface roughness of a titanium prosthetic implant before the polishing step showing Ra value of 0.304.

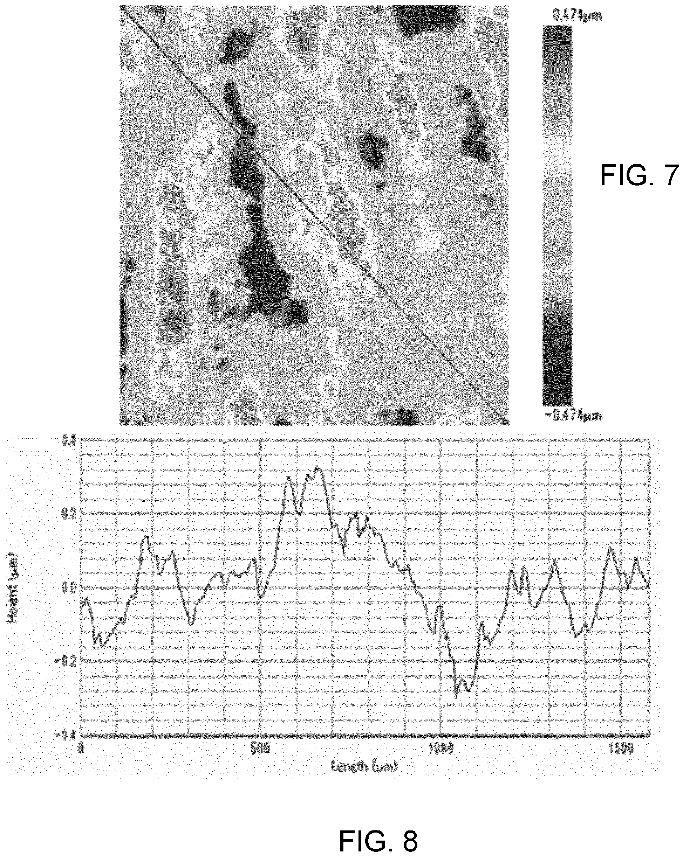

After the polishing step the Ra value is reduced to 0.014, as indicated by the data in Figures 7 and 8, thus providing a clear reduction in surface roughness.

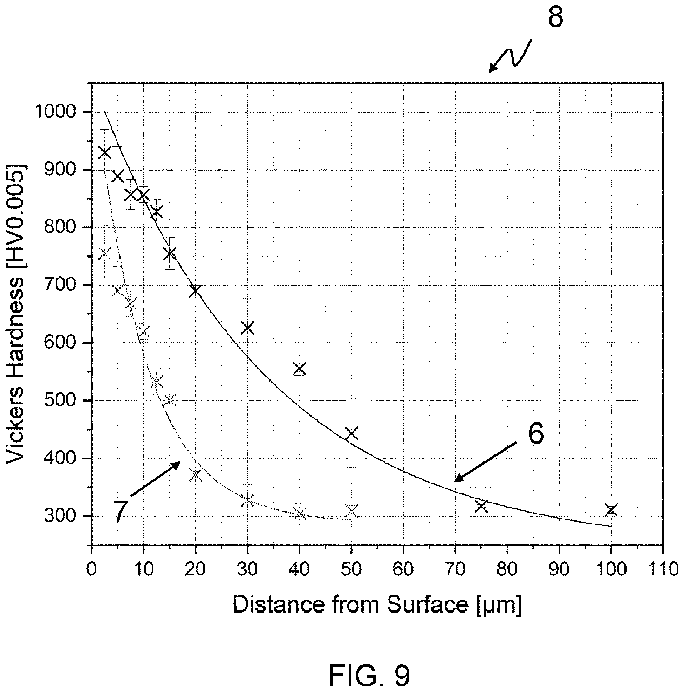

Figure 9 is a plot showing cross-sectional hardness profiles of titanium prosthetic implants according to some embodiments of the invention.

Vickers Hardness values were obtained based on the mean value of three indentations set with a load of 5g and a dwell time of 10s. Indentations were placed in specific distances measured orthogonally from the external surface. The obtained Hardness profiles relate thereby to different case hardening conditions.

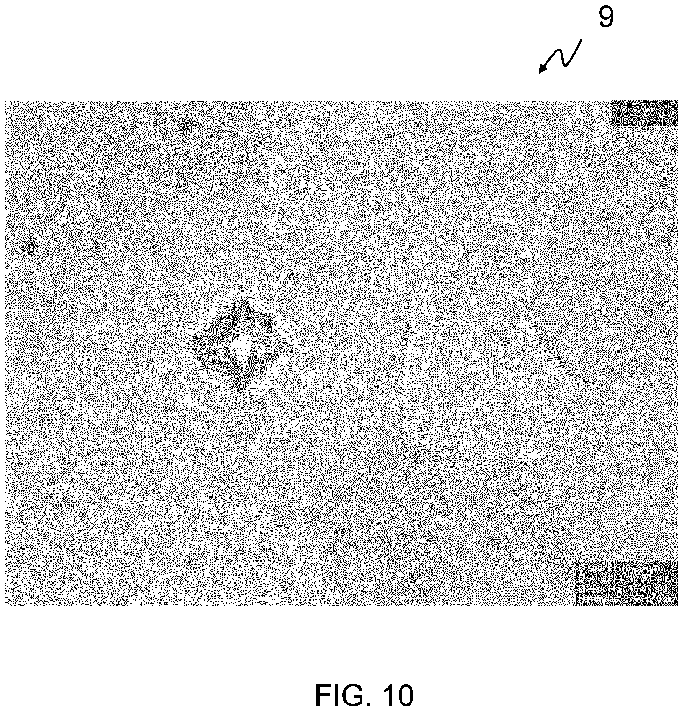

Figure 10 is a microscopy image of a metallic surface of a titanium prosthetic implant, according to some embodiments of the invention, following a hardness test.

Vickers Hardness indentation was set with a load of 50g and a dwell time of 10s top-down into the case hardened and polished surface.

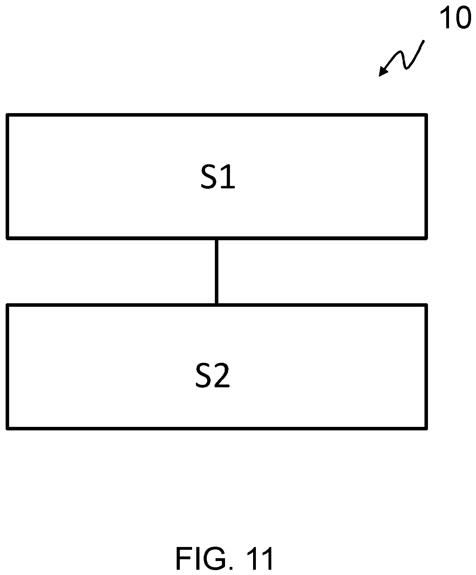

Figure 11 shows a flow chart of a method 10 of reducing wear and abrasion during use of a titanium metal or a titanium metal alloy prosthetic implant system according to second aspect of the invention.

The method 10 comprises the steps of:
- S1 case hardening the titanium or a titanium metal alloy prosthetic implant;
- S2 polishing the case-hardened titanium metal or a titanium metal alloy prosthetic implant by electropolishing.

The polishing or electropolishing is achieved by applying a voltage of at least 40 V, such as between 40 V and 60 V, for example between 40 V and 50 V, between an electrode and a counter electrode for one or more pre-determined periods of time.

The pre-determined period of time could be between 10 µs to 10 s.

The application of voltage may occur in separate steps, having different pre-determined periods of time.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is possible and advantageous.

## Claims

1. A titanium metal or titanium metal alloy prosthetic implant, such as a joint replacement implant, said prosthetic implant comprising a metallic surface layer having a thickness between 5 and 50 µm, said metallic surface layer having a surface hardness above 700 HV_{0.05}.

2. A titanium metal or a titanium metal alloy prosthetic implant according to claim 1, wherein said metallic surface layer has a surface roughness in the range between 0.1 and 0.01 Ra.

3. A titanium metal or a titanium metal alloy prosthetic implant according to any of the preceding claims 1-2, wherein said metallic surface layer has a hardness factor (HF) of 1 (HF1) as detectable through a VDI 3198 indentation test.

4. A titanium metal or a titanium metal alloy prosthetic implant according to any of the preceding claims 1-3, wherein said metallic surface layer is produced by case hardening of said titanium metal or a titanium metal alloy prosthetic implant.

5. A titanium metal or a titanium metal alloy prosthetic implant according to any of the preceding claims 1-4, wherein said case hardening is achieved by exposure to heat in a reactive atmosphere such as in presence of COz or NH₃ gas.

6. A titanium metal or a titanium metal alloy prosthetic implant according to any of the preceding claims 1-5, wherein said surface roughness is achieved by an electropolishing process applying a voltage of at least 40 V, such as between 40 V and 60 V.

7. A titanium metal or a titanium metal alloy prosthetic implant according to any of the preceding claims 1-6, wherein said prosthetic implant is a titanium joint replacement.

8. A titanium metal or a titanium metal alloy prosthetic implant system comprising said titanium metal or a titanium metal alloy prosthetic implant according to any preceding claims 1-7 and a polymer-based bearing component.

9. A method of reducing wear and abrasion during use of a titanium metal or a titanium metal alloy prosthetic implant system according to claim 8, said method comprising:
- case hardening said titanium or a titanium metal alloy prosthetic implant;
- polishing said case hardened titanium metal or a titanium metal alloy prosthetic implant by electropolishing applying a voltage of at least 40 V, such as between 40 V and 60 V.

10. A method according to claim 9, wherein said polishing comprises:
- applying a voltage of at least 40 V, such as between 40V and 60V between an electrode and a counter electrode for one or more pre-determined periods of time.
